# EUROPÄISCHE PATENTSCHRIFT

(11) **EP 0 476 400 B1**
(45) Veröffentlichungstag und Bekanntmachung des Hinweises auf die Patenterteilung: **21.01.1998**
(21) Anmeldenummer: 91114688.4
(22) Anmeldetag: 31.08.1991
(51) Int. Cl.: C07C 7/17, C07C 29/04, C07C 69/04

(54) **Verfahren zur Abtrennung von Cyclohexen aus Gemischen mit Benzol und Cyclohexan**
Method of separating cyclohexene from mixtures with benzene and cyclohexane
Procédé de séparation de cyclohexene des mélanges avec benzènes et cyclohexane

(30) Priorität: 18.09.1990 DE 4029485
(43) Veröffentlichungstag der Anmeldung: 25.03.1992
(73) Patentinhaber: BASF Aktiengesellschaft, 67063 Ludwigshafen (DE)
(72) Erfinder: Fischer, Rolf, Dr., W-6900 Heidelberg (DE); Gosch, Hans-Juergen, Dr., W-6702 Bad Duerkheim (DE); Lermer, Helmut, Dr., W-6700 Ludwigshafen (DE); Pinkos, Rolf, Dr., W-6702 Bad Duerkheim (DE); Weyer, Hans-Juergen, Dr., W-6800 Mannheim 1 (DE)

(56) Entgegenhaltungen:
- EP-A- 0 123 713
- EP-A- 0 162 475
- EP-A- 0 340 827
- EP-A- 0 341 163
- PATENT ABSTRACTS OF JAPAN vol. 11, no. 317 (C-452)(2764) 15 Oktober 1987, & JP-62 103 033
- PATENT ABSTRACTS OF JAPAN vol. 2, no. 93 (C-78)(1679) 29 Juli 1978, & JP-A-53 059649

## Beschreibung

Aus der EP-A 341 163, der EP-A 162 475 und der japanischen Patentanmeldung 12 54 634 ist bekannt, daß bei der Umsetzung von Cyclohexen mit Carbonsäure und Wasser in Gegenwart von ZSM-Zeolithen, Alumosilikaten bzw. starksauren Ionenaustauschern Wasser, Gemische aus Cyclohexanol und Cyclohexylestern erhalten werden. Angaben zum Auftreten von Nebenprodukten, d.h. zur Selektivität bei der Umsetzung des Cyclohexens mit Carbonsäuren und gegebenenfalls Wasser, sind nicht zu entnehmen.

Cyclohexen läßt sich ausgehend von Benzol oder Cyclohexan herstellen: Bei der partiellen Hydrierung von Benzol z.B. an Ruthenium-Katalysatoren (vgl. DE-A 22 21 137) oder bei der katalytischen Dehydrierung von Cyclohexan erhält man jedoch kein reines Cyclohexen, sondern Gemische aus Cyclohexen, Benzol und Cyclohexan wechselnder Zusammensetzung. Die Abtrennung des Cyclohexens aus diesen Gemischen, z.B. durch Extraktivdestillation oder Azeotropdestillation, stellt einen sehr aufwendigen Reaktionsschritt dar.

Bei der Umsetzung von Cyclohexen mit Wasser in Gegenwart von starksauren Katalysatoren, z.B. Zeolithen, werden lediglich Cyclohexanol-Ausbeuten von maximal 15 % erzielt (siehe EP 341 163, Vergleichsbeispiel a). Daher ist diese Methode für die Abtrennung von Cyclohexen aus seinen Gemischen mit Benzol und Cyclohexan weniger geeignet.

Es ist bekannt, Cyclohexen in Gemischen aus Cyclohexen, Benzol und Cyclohexan in Gegenwart von Homogenkatalysatoren, beispielsweise gemäß EP-A 340 827 in Gegenwart von Schwefelsäure oder gemäß EP-A 123 713 in Gegenwart eines Gemisches aus einer Heteropolysäure und einer aromatischen Sulfonsäure, zu hydratisieren. Die Abtrennung des Cyclohexanols aus dem Reaktionsgemisch und die Reinigung des Produktes wird aber durch die Gegenwart des Homogenkatalysators erschwert.

Es bestand die technische Aufgabe, ein wirkungsvolleres Verfahren zu entwickeln, das die Abtrennung von Cyclohexen aus Gemischen mit Benzol und Cyclohexan mit der Herstellung von Cyclohexanol und Cyclohexylcarbonsäureestern verbindet. Eine weitere Aufgabe bestand darin, die Abtrennung des Cyclohexens mit möglichst hoher Selektivität durchzuführen und die Bildung von Nebenprodukten zu minimieren.

Diese Aufgabe wird gelöst durch ein Verfahren zur Abtrennung von Cyclohexen aus seinen Gemischen mit Benzol und Cyclohexan, indem man diese Gemische in Gegenwart von festen starksauren Katalysatoren mit Carbonsäuren und Wasser in Berührung bringt, wobei man
(a) die Reaktion in flüssiger Phase bei einer Temperatur im Bereich von 20 bis 180°C und einem Molverhältnis von Carbonsäure zu Wasser im Bereich von 3 : 15 bis 30 : 0,1 durchführt, und gegebenenfalls nach der Reaktion mindestens soviel Wasser zusetzt, daß nach der Reaktion eine Trennung in eine wäßrige Phase und eine organische Phase erfolgt,
(b) das in (a) erhaltene flüssige Reaktionsgemisch nach Abtrennen des festen Katalysators in eine wäßrige und eine organische Phase trennt,
(c) die wäßrige Phase ggf. nach Extraktion mit Gemischen aus Benzol, Cyclohexen und/oder Cyclohexan in die Stufe (a) zurückführt, und
(d) die in der organischen Phase enthaltenen Cyclohexylcarbonsäureester verseift und Cyclohexanol gewinnt.

Die erfindungsgemäß eingesetzten Gemische aus Cyclohexen, Benzol und Cyclohexan sind durch partielle Hydrierung von Benzol oder durch Dehydrierung von Cyclohexan herstellbar. Die Zusammensetzung dieser gegebenenfalls durch geeignete Methoden wie z. B. Destillation vorbehandelten Gemische beträgt beispielsweise 0,1 bis 80 Gew.-% Cyclohexen, 0,1 bis 90 Gew,-% Benzol und 5 bis 90 Gew.-% Cyclohexan. Bevorzugte Gemische enthalten 3 bis 50 Gew.-% Cyclohexen, 10 bis 80 Gew.-% Benzol und 5 bis 50 Gew.-% Cyclohexan.

Als Carbonsäuren können aliphatische, cycloaliphatische, aromatische oder araliphatische Carbonsäuren verwendet werden. Die Carbonsäuren können eine oder mehrere Carboxylgruppen enthalten. Beispiele für aliphatische Carbonsäuren sind solche mit einem bis achtzehn Kohlenstoffatomen wie z. B. Ameisensäure, Essigsäure, Propionsäure, n- und i-Buttersäure, Oxalsäure, Glutarsäure, Adipinsäure. Die aliphatischen Carbonsäuren können Halogenatome wie Chlor oder Fluor enthalten. Beispiele hierfür sind Monochloressigsäure, Trichloressigsäure oder Trifluoressigsäure. Beispiele für cycloaliphatische Carbonsäüren sind Cyclohexancarbonsäure und 1.4-Cyclohexandicarbonsäure. Beispiele für aromatische und araliphatische Carbonsäuren sind Phenylessigsäure, Benzoesäure und Terephthalsäure. Vorteilhaft verwendet man Fettsäuren mit 1 bis 8 Kohlenstoffatomen.

Besonders bevorzugte Carbonsäuren sind Ameisensäure, Essigsäure und Propionsäure.

Das Molverhältnis von Cyclohexen zu Carbonsäure beträgt zweckmäßig 1 : 0,5 bis 1 : 30, insbesondere 1 : 2 bis 1 : 6.

Das Molverhältnis von Cyclohexen zu Wasser beträgt vorteilhaft 1 : 0,1 bis 1 : 15, insbesondere 1 : 2 bis 1 : 6.

Hieraus errechnet sich ein Molverhältnis von Carbonsäure zu Wasser im Bereich von 3 : 15 bis 30 : 0,1, vorzugsweise 3 : 6 bis 30 : 1.

Als feste starksaure Katalysatoren sind z. B. starksaure Ionentauscher, Silikate oder natürliche oder synthetisch hergestellte Zeolithe geeignet. Besonders bevorzugt sind Zeolithe.

Bevorzugte stark saure lonentauscher sind z.B. vernetztes Polystyrol mit Sulfonsäuregruppen.

Geeignet sind beispielsweise Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe von Zeolithen gehören auch die sogenannten "ultrastabilen" Zeolithe des Faujasittyps, d.h. dealuminierte Zeolithe.

Besonders vorteilhaft sind Zeolithe mit Pentasilstruktur wie ZS M-5, ZS M-11 und ZB M-10-Zeolithe. Diese haben als Grundbaustein einen aus SiO₂-Tetraedern aufgebauten Fünfring gemeinsam. Sie sind durch ein hohes SiO₂/Al₂O₃-Verhältnis gekennzeichnet sowie durch Porengrößen, die zwischen denen der Zeolithe vom Typ A und denen vom Typ X oder Y liegen.

Geeignete Zeolithe können eine unterschiedliche chemische Zusammensetzung aufweisen. Es handelt sich um Alumino-, Boro-, Eisen-, Beryllium-, Gallium-, Chrom-, Arsen-, Antimon- und Wismutsilikatzeolith oder deren Gemische sowie Alumino-, Boro-, Gallium-und Eisengermanatzeolithe oder deren Gemische. Insbsondere eignet sich die Alumino-, Boro- und Eisensilikatzeolithe mit Pentasilstruktur für das erfindungsgemäße Verfahren. Der Aluminiumsilikatzeolith wird z.B. aus einer Aluminiumverbindung, vorzugsweise Al(OH)₃ oder Al₂(SO₄)₃ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 200°C unter autogenem Druck hergestellt. Auch gehören hierzu die isotaktischen Zeolithe. Die erhaltenen Aluminosilikatzeolithe enthalten je nach Wahl der Einsatzstoffmengen ein SiO₂/Al₂O₃-Verhältnis von 10 bis 40 000. Auch lassen sich derartige Aluminosilikatzeolithe in etherischem Medium wie Diethylenglykoldimethylether, in alkoholischem Medium wie Methanol bzw. 1,4-Butandiol oder Wasser synthetisieren.

Der Borosilikatzeolith wird z.B. bei 90 bis 200°C unter autogenem Druck synthetisiert, indem man eine Borverbindung z.B. H₃BO₃, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch gehören hierzu die isotaktischen Zeolithe. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise Fe₂(SO₄)₃ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220°C unter autogenem Druck.

Die so hergestellten Alumino-, Boro- und Eisensilikatzeolithe können nach Ihrer Isolierung, Trocknung bei 100 bis 160°C, vorzugsweise 110°C und Calcinierung bei 450 bis 550°C, vorzugsweise 500°C, mit einem Bindemittel im Verhältnis 90:10 bis 40:60 Gew.-% zu Strängen oder Tabletten verformt werden. Als Bindemittel eignen sich diverse Aluminiumoxide, bevorzugt Böhmit, amorphe Aluminosilikate mit einem SiO₂/Al₂O₃-Verhältnis von 25:75 bis 90:5, bevorzugt 75:25, Siliciumdioxid, bevorzugt hochdisperses SiO₂, Gemische aus hochdispersem SiO₂ und hochdispersem Al₂O₃, TiO₂, ZrO₂ sowie Ton. Nach der Verformung werden die Extrudate oder Preßlinge bei 110°C/16 h getrocknet und bei 500°C/16 h calciniert.

Man erhält auch vorteilhafte Katalysatoren, wenn der isolierte Alumino- bzw. Borosilikatzeolit direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/N₂-Gemisch bei 400 bis 500°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, kann es vorteilhaft sein, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4. bis 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Fr, Yb und U eingesetzt: Der Gehalt an solchen Modifizierungsmittel beträgt im allgemeinen 0,001 bis 20 Gew.-%, insbesondere 0,01 bis 5 Gew.-%.

Zweckmäßigerweise führt man die Dotierung so durch, daß man z.B. den verformten Zeolithen in einem Steigrohr vorlegt und bei 20 bis 100°C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Cu(NO₃)₂ x 3 H₂O oder Ni(NO₃)₂ x 6 H₂O oder Ce(NO₃)₃ x 6 H₂O oder La(NO₃)₂ x 6 H₂O oder Cs₂CO₃ in Wasser löst. Mit dieser Lösung wird der verformte und der unverformte Zeolith eine gewisse Zeit, ca. 30 Minuten, getränkt. Die eventuell überstehende Lösung wird am Rotationsverdampfer von Wasser befreit. Danach wird der getränkte Zeolith bei ca. 150°C getrocknet und bei ca. 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne verlegt und darüber z.B. eine wäßrige Ni(NO₃)₂-Lösung oder ammoniakalische Pd(NO₃)₂-Lösung bei leicht erhöhter Temperatur zwischen 30 und 80°C im Kreislauf 15 bis 20 h leitet. Danach wird mit Wasser ausgewaschen, bei ca. 150°C getrocknet und bei ca. 550°C calciniert. Bei manchen metalldotierten Zeolithen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft z.B. so vor, daß man Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500 °C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80°C mit einer 3 bis 25 gew.-%igen, insbesondere 12 bis 20 gew.-%igen wäßrigen Salzsäure. Anschließend wird der so behandelte Zeolith mit Wasser gewaschen, getrocknet und bei 400°C bis 500°C calciniert.

Eine besondere Ausführungsform für die Säurebehandlung besteht darin, daß man das zeolithische Material vor seiner Formung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen, des zeolithischen Materials wird dieses zweckmäßig, z.B. bei Temperaturen von 100 bis 160°C, getrocknet und bei Temperaturen von im allgemeinen 450 bis 600°C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50 bis 90°C, vorzugsweise 60 bis 80°C, über einen Zeitraum von 0,5 bis 5, vorzugsweise mit 12 bis 20 gew.-%iger Salzsäure, behandelt. Anschließend wird das zeolithische Material im allgemeinen ausgewaschen und zweckmäßig, z.B. bei Temperaturen von 100 bis 160°C, getrocknet und bei Temperaturen von im allgemeinen 450 bis 600°C calciniert. Einer HF-Behandlung kann sich auch eine HCl-Behandlung anschließen.

Die Katalysatormenge beträgt, bezogen auf das im Benzol/Cyclohexen/Cyclohexan-Gemisch enthaltene Cyclohexen z.B. 10 Gew.-% bis 2000 Gew.-%, insbesondere 30 Gew.-% bis 600 Gew.-%.

Die erfindungsgemäße Umsetzung wird bei erhöhten Temperaturen z.B. von 20 bis 180°C, insbesondere 70 bis 140°C durchgeführt.

Die Umsetzung wird in der Flüssigphase bei Normaldruck oder erhöhtem Druck durchgeführt. Der Druck muß so hoch gewählt werden, daß das Reaktionsgemisch flüssig bleibt.

die Verweilzeiten liegen z. B. bei etwa 15 Minuten bis 6 Stunden, insbesondere einer bis 3 Stunden.

Die Umsetzung läßt sich diskontinuierlich oder kontinuierlich durchführen, wobei der Katalysator suspendiert oder fest angeordnet sein kann.

Sollte nach der Reaktion keine Phasentrennung erfolgen, so kann man nach der Reaktion gegebenenfalls soviel Wasser zusetzen, daß eine Trennung in eine wäßrige Phase und eine organische Phase erfolgt.

Die Phasentrennung, die erfindungsgemäß nach der Abtrennung des Katalysators erfolgt, kann drucklos oder unter Druck, bei Temperaturen von 0°C bis 180°C, insbesondere 20°C bis 140°C durchgeführt werden.

Erfindungsgemäß wird die wäßrige Phase, die Carbonsäure, Wasser, Cyclohexen, Benzol, Cyclohexan, Cyclohexylester und Cyclohexanol enthält, in die Synthesestufe zurückgeführt. Hierbei kann es sinnvoll sein, den Gehalt der wäßrigen Phase an Cyclohexylcarbonsäureester und Cyclohexanol durch Extraktion mit Gemischen aus Benzol, Cyclohexan und/oder Cyclohexan weiter abzusenken. Hierdurch wird die im Kreis geführte Menge an Wertprodukten verringert.

Die Gemische aus Benzol, unumgesetztem Cyclohexen und Cyclohexan werden zweckmäßig aus der organischen Phase destillativ abgetrennt. Sie werden vorteilhaft zur weiteren Abreicherung des Cyclohexens in einer zweiten Reaktionsstufe erneut mit Carbonsäure umgesetzt. Bemerkenswert ist, daß auch noch bei niedrigen Cyclohexen-Gehalten im Gemisch mit Benzol und Cyclohexan verglichen mit der Hydratisierung von Cyclohexen, bemerkenswert hohe Cyclohexen-Umsätze erreicht werden.

Je nach gewünschtem Abreicherungsgrad des Cyclohexens wendet man zusätzlich eine dritte und gegebenenfalls vierte Reaktionsstufe an.

Der in der organischen Phase nach der Abtrennung von Benzol, Cyclohexen und Cyclohexan enthaltene Cyclohexylcarbonsäureester wird verseift und Cyclohexanol gewonnen.

Es war nicht vorauszusehen, daß die Abtrennung von Cyclohexen aus Gemischen mit Benzol und Cyclohexan mit hoher Selektivität ablaufen würde:

Es ist nämlich bekannt, daß sich Benzol mit Cyclohexen in Gegenwart von Zeolithen zu Cyclohexylbenzol umsetzen kann:

(EP-A 338.734, Japanische Patentanmeldung 59/137 426, Dokl. Akad. Nauk SSSR 237(1), 164-7 (1977), U.S.Patent 4.122.125, 4.206.082, 4.217.248). Zu erwarten war daher, daß unter den Reaktionsbedingungen der Carbonsäure-Anlagerung an Cyclohexen auch größere, selektivitätsmindernde Mengen an Cyclohexylbenzol entstehen würden.

Aus EP 162 475, Seiten 19 bis 20, ist weiterhin bekannt, daß Carbonsäuren in Kombination mit Zeolithen die Hydratisierung von Cyclohexen besser katalysieren als Zeolithe allein. Es war daher zu erwarten, daß die literaturbekannte Nebenreaktion der Hydratisierung (z. B. DE-PS 3 441 072, Beispiel 5, EP 162 475, Seite 8, Zeile 15 bis 17, JP 61/249 945), die ebenfalls sauer katalysierte Anlagerung von Cyclohexanol an Cyclohexen zu Dicyclohexylether, verstärkt in Erscheinung treten würde. Dies um so mehr, als bei der Anlagerung von Carbonsäuren an Cyclohexen in Gegenwart von Wasser zum Teil größere Mengen an Cyclohexanol als bei der Hydratisierung von Cyclohexen im Reaktionsgemisch vorliegen.

Weiterhin ist bekannt, daß Carbonsäuren in Gegenwart von Zeolithen Benzol zu acylieren vermögen (J. Org. Chem. 1986, 51, 2128 bis 2130). Es war daher zu erwarten, daß als Nebenprodukte Acylbenzole auftreten würden.

Im folgenden bedeutet Anol Cyclohexanol.

### Beispiel 1 a

2,56 g eines Gemisches aus 32 Gew.-% Cyclohexen, 60 Gew.-% Benzol und 8 Gew.-% Cyclohexan sowie 0,85 g Wasser, 2,82 g Essigsäure und 0,5 g pulverförmigem Zeolith (H-ZSM 11) wurden in einem 10 ml Autoklaven gerührt und mit Argon gespült. Dann wurde auf 120°C erhitzt. Der Eigendruck betrug 2,8 bar. Nach zwei Stunden wurde auf Raumtemperatur abgekühlt. Die zwei flüssigen Phasen wurden mit 12 g Aceton homogenisiert. Das Reaktionsgemisch wurde nach Abfiltrieren des Zeoliths gaschromatographisch (Kapillarsäule, innerer Standard) analysiert. Es enthielt 41,9 % Cyclohexylacetat und 22 % Cyclohexanol (bezogen auf eingesetztes Cyclohexen). Die Gehalte an Dicyclohexylether, Cyclohexylbenzol und Acetylbenzol lagen unter der GC-Nachweisgrenze. Weitere Nebenprodukte wurden ebenfalls nicht gefunden.

### Beispiel 1 b

2,56 g eines Gemisches aus 10 Gew.-% Cyclohexen, 77 Gew.-% Benzol und 13 Gew.-% Cyclohexan sowie 0,85 g Wasser, 0,86 g Essigsäure und 0,5 g pulverförmigem Zeolith (H-ZSM 11) wurden, wie in Beispiel 1 a beschrieben, umgesetzt und analysiert. Das Reaktionsgemisch enthielt 32,9 % Cyclohexylester und 13,6 % Cyclohexanol (bezogen auf eingesetztes Cyclohexen). Die Gehalte an Dicyclohexylether, Cyclohexylbenzol und Acetylbenzol lagen unter der GC-Nachweisgrenze.

### Beispiel 1 c

2,56 g eines Gemisches aus 3 Gew.-% Cyclohexen, 83 Gew.-% Benzol und 14 Gew.-% Cyclohexan sowie 0,078 g Wasser, 0,26 g Essigsäure und 0,5 g pulverförmigem Zeolith (H-ZSM 11) wurden wie in Beispiel 1 a umgesetzt. Der Reaktoraustrag enthielt 39,2 % Cyclohexylester und 8,7 % Cyclohexanol (bezogen auf eingesetztes Cyclohexen). Die Gehalte an Dicyclohexylether, Cyclohexylbenzol und Acetylbenzol lagen unter der GC-Nachweisgrenze.

### Beispiel 2

2,56 g eines Gemisches an 10 Gew.-% Cyclohexen, 77 Gew.-% Benzol und 13 Gew.-% Cyclohexan sowie 0,85 g Wasser und 2,82 g Essigsäure und 0,5 g pulverförmigem Zeolith (H-ZSM 11) wurden wie in Beispiel 1 a umgesetzt. Der Reaktoraustrag enthielt 45,6 % Cyclohexylester und 21,5 % Cyclohexanol (bezogen auf eingesetztes Cyclohexen).

### Beispiel 3

Das in Beispiel 1 c angegebene Gemisch an Benzol, Cyclohexen und Cyclohexan wurde mit 0,26 g Wasser, 0,84 g Essigsäure und 0,5 g pulverförmigem Zeolith (H-ZSM 11) wie in Beispiel 1 a beschrieben umgesetzt. Der Reaktionsaustrag enthielt 36,1 % Cyclohexylester und 12,5 % Cyclohexanol (bezogen auf eingesetztes Cyclohexen).

### Beispiel 4

Wie in Beispiel 1 a beschrieben wurden 5,12 g eines Gemisches aus 32 Gew.-% Cyclohexen, 60 Gew.-% Benzol und 8 Gew.-% Cyclohexan sowie 0,36 g Wasser, 0,92 g Ameisensäure und 1 g pulverförmigem Zeolith (H-ZSM 11) auf 110°C erhitzt und wie dort angegeben aufgearbeitet. Der Reaktionsaustrag enthielt laut gaschromatographischer Analyse 81,4 % Cyclohexylformiat und 2,9 % Cyclohexanol (bezogen auf eingesetztes Cyclohexen). Die Gehalte am Dicyclohexylether, Cyclohexylbenzol und Benzaldehyd lagen unter der GC-Nachweisgrenze, weitere Nebenprodukte wurden ebenfalls nicht gefunden.

### Beispiel 5

2,56 g eines Gemisches aus 32 Gew.-% Cyclohexen, 60 Gew.-% Benzol und 8 Gew.-% Cyclohexan sowie 0,85 g Wasser, 3,48 g Propionsäure und 0,5 g pulverförmigem Zeolith (H-ZSM 11) wurden wie in Beispiel 1 a beschrieben umgesetzt. Der Reaktionsaustrag enthielt 32,8 % Cyclohexylpropionat und 12,9 % Cyclohexanol (bezogen auf eingesetztes Cyclohexen).

### Beispiel 6

5,1 g eines Gemisches aus 32 Gew.-% Cyclohexen, 60 Gew.-% Benzol und 8 Gew.-% Cyclohexan sowie 5,60 g Essigsäure und 1 g pulverförmigem Zeolith (H-ZSM 11) wurden mit a) 1,0, b) 1,1, c) 1,7, d) 2,0 und e) 2,3 Mol Wasser/Mol Cyclohexen versetzt und nach Spülen mit Argon 2 Stunden auf 120°C erhitzt. Nach Abkühlen auf Raumtemperatur wurden die organische, die wäßrige und die Zeolithphase voneinander getrennt. Die Ausbeuten an Anol und Ester wurden gaschromatographisch, die Essigsäurewerte potentiometrisch ermittelt (Tab. 1).

### Beispiel 7

5,1 g eines Gemisches aus 32 Gew.-% Cyclohexen, 60 Gew.-% Benzol und 8 Gew.-% Cyclohexan sowie 4,3 g Ameisensäure und 1 g pulverförmiger Zeolith (H-ZSM 11) wurden mit a) 0,7, b) 1,1, c) 1,6 und d) 2,2 Mol Wasser/Mol Cyclohexen versetzt und nach Spülen mit Argon 2 Stunden auf 110°C erhitzt. Nach Abkühlen auf Raumtemperatur wurden die organische, die wäßrige und die Zeolithphase voneinander getrennt. Die Ausbeuten an Anol und Ester wurden gaschromatographisch, die Ameisensäurewerte potentiometrisch ermittelt (Tab. 1).

**Tabelle 1**

| Versuchsergebnisse zu den Beispielen 6 und 7. | | | | | | | | | |
|---|---|---|---|---|---|---|---|---|---|
| Beispiel | organische Phase Ester Anol Säure (Mol%) 1) | | | wäßrige Phase Ester Anol Säure (Mol%) 1) | | | Zeolith Ester Anol Säure (Mol%) 1) | | |
| 6 a | 39,8 | 8,5 | 59 | 2,6 | 1,0 | 3 | 5,0 | 2,2 | 37 |
| 6 b | 38,0 | 8,3 | 51 | 2,8 | 1,2 | 16 | 4,8 | 2,4 | 33 |
| 6 c | 33,2 | 8,8 | 34 | 2,9 | 2,5 | 33 | 3,2 | 2,5 | 31 |
| 6 d | 29,6 | 7,7 | 26 | 2,4 | 2,6 | 43 | 2,3 | 2,3 | 31 |
| 6 e | 30,5 | 8,3 | 23 | 2,4 | 2,9 | 45 | 1,5 | 2,6 | 31 |
| | | | | | | | | | |
| 7 a | 67,6 | 3,8 | 20 | 1,8 | 0,2 | 21 | 6,2 | 0,8 | 54 |
| 7 b | 58,4 | 4,5 | 15 | 1,8 | 0,5 | 29 | 8,3 | 1,2 | 51 |
| 7 c | 58,6 | 5,4 | 7 | 1,6 | 0,9 | 36 | 3,6 | 1,1 | 48 |
| 7 d | 51,8 | 5,6 | 7 | 1,8 | 1,0 | 48 | 2,8 | 1,3 | 44 |

| | | | | | | | | | |
|---|---|---|---|---|---|---|---|---|---|
| 1) Bezogen auf eingesetzes Cyclohexen | | | | | | | | | |

### Beispiel 8

5,1 g eines Gemisches aus 32 Gew.-% Cyclohexen, 60 Gew.-% Benzol und 8 Gew.-% Cyclohexan sowie 5,60 g Essigsäure und 1 g pulverförmigem Zeolith (H-ZSM 11) wurden mit 2,3 Mol Wasser/Mol Cyclohexen versetzt und nach Spülen mit Argon 2 Stunden auf 120°C erhitzt. Nach Abkühlen auf Raumtemperatur wurde die organische von den übrigen Phasen getrennt. Die Wasser/Zeolithphase wurde zweimal mit 5,5 g des angegebenen Benzol/Cyclohexen/Cyclohexan-Gemisches extrahiert. Der Gehalt an Anol und Ester der resultierenden organischen Phasen wurde gaschromatographisch bestimmt (Tab. 2).

**Tabelle 2**

| Abreicherung des Cyclohexylester- und Anolgehaltes in der wäßrigen Phase (Mol-%, bezogen auf eingesetztes Cyclohexen) | | | | | |
|---|---|---|---|---|---|
| org. Phase nach der Reaktion | | org. Phase nach der ersten Extraktion | | org. Phase nach der zweiten Extraktion | |
| Ester | Anol | Ester | Anol | Ester | Anol |
| 31,3 | 8,6 | 4,2 | 3,6 | 0,4 | 1,5 |

### Beispiel 9

2,56 g eines Gemisches aus 32 Gew.-% Cyclohexen, 60 Gew.-% Benzol und 8 Gew.-% Cyclohexan sowie 4,8 g Valeriansäure und 50 g Ionentauscher auf Basis vernetztem Polystyrol mit Sulfonsäuregruppen wurden in einem 10 ml Autoklaven gerührt und mit Argon gespült. Dann wurde auf 1200C erhitzt. Nach zwei Stunden wurde auf Raumtemperatur abgekühlt. Die flüssige Phase wurde mit 12 g Aceton verdünnt und nach Abfiltrieren des Ionentauschers gaschromatographisch (Kapillarsäule, innerer Standard) analysiert. Sie enthielt 55,5 % Cyclohexylvaleriat (bezogen auf eingesetztes Cyclohexen). Die Gehalte an Dicyclohexylether, Cyclohexylbenzol und n-Butyl-phenylketon lagen unter der GC-Nachweisgrenze. Weitere Nebenprodukte wurden ebenfalls nicht gefunden.

### Beispiel 10

2,56 g eines Gemisches aus 32 Gew.-% Cyclohexen, 60 Gew.-% Benzol und 8 Gew.-% Cyclohexan sowie 2,82 g Essigsäure, 0,14 g Wasser und 64,1 g Ionentauscher auf Basis vernetztem Polystyrol mit Sulfonsäuregruppen wurden in einem 10 ml Autoklaven gerührt und mit Argon gespült. Dann wurde auf 120°C erhitzt. Nach zwei Stunden wurde auf Raumtemperatur abgekühlt. Die flüssige Phase wurde mit 12 g Aceton verdünnt und nach Abfiltrieren des Ionentauschers gaschromatographisch (Kapillarsäule, innerer Standard) analysiert. Sie enthielt 35,6 % Cyclohexylacetat und 3,6 % Cyclohexanol (bezogen auf eingesetztes Cyclohexen). Die Gehalte an Dicyclohexylether, Cyclohexylbenzol und Acetylbenzol lagen unter der GC-Nachweisgrenze.

### Beispiel 11

2,56 g eines Gemisches aus 32 Gew.-% Cyclohexen, 60 Gew.-% Benzol und 8 Gew.-% Cyclohexan sowie 5,4 g n-Hexansäure und 50 g Ionentauscher auf Basis vernetztem Polystyrol mit Sulfonsäuregruppen wurden in einem 10 ml Autoklaven gerührt und mit Argon gespült. Dann wurde auf 120°C erhitzt. Der Eigendruck betrug 2 bar. Nach zwei Stunden wurde auf Raumtemperatur abgekühlt. Die flüssige Phase wurde mit 12 g Aceton verdünnt und nach Abfiltrieren des Ionentauschers gaschromatographisch (Kapillarsäule, innerer Standard) analysiert. Sie enthielt 58,2 % Cyclohexylhexanoat und 1,5 % Cyclohexanol (bezogen auf eingesetztes Cyclohexen). Die Gehalte an Dicyclohexylether, Cyclohexylbenzol und n-Pentyl-phenylketon lagen unter der GC-Nachweisgrenze.

### Beispiel 12

2,56 g eines Gemisches aus 32 Gew.-% Cyclohexen, 60 Gew.-% Benzol und 8 Gew.-% Cyclohexan sowie 2,16 g Ameisensäure und 0,5 g pulverförmigem Zeolith (H-ZSM 11) wurden in einem 10 ml Autoklaven gerührt und mit Argon gespült. Dann wurde auf 80°C erhitzt. Nach zwei Stunden wurde auf Raumtemperatur abgekühlt. Die flüssige Phase wurde mit 12 g Aceton verdünnt und nach Abfiltrieren des Zeoliths gaschromatographisch (Kapillarsäule, innerer Standard) analysiert. Sie enthielt 90,4 % Cyclohexylformiat und 1,3 % Cyclohexanol (bezogen auf eingesetztes Cyclohexen). Die Gehalte an Dicyclohexylether, Cyclohexylbenzol und Benzaldehyd lagen unter der GC-Nachweisgrenze.

### Beispiel 13

2,05 g eines Gemisches aus 40 Gew.-% Cyclohexen, 20 Gew.-% Benzol und 40 Gew.-% Cyclohexan sowie 2,16 g Ameisensäure, 0,18 g Wasser und 0,5 g pulverförmigem Zeolith (H-ZSM 11) wurden in einem 10 ml Autoklaven gerührt und mit Argon gespült. Dann wurde auf 100°C erhitzt. Nach zwei Stunden wurde auf Raumtemperatur abgekühlt. Die flüssige Phase wurde mit 12 g Aceton verdünnt und nach Abfiltrieren des Zeoliths gaschromatographisch (Kapillarsäule, innerer Standard) analysiert. Sie enthielt 78,1 % Cyclohexylformiat und 4,9 % Cyclohexanol (bezogen auf eingesetztes Cyclohexen). Die Gehalte an Dicyclohexylether, Cyclohexylbenzol und Benzaldehyd lagen unter der GC-Nachweisgrenze.

### Beispiel 14

2,56 g eines Gemisches aus 32 Gew.-% Cyclohexen, 60 Gew.-% Benzol und 8 Gew.-% Cyclohexan sowie 2,82 g Essigsäure, 0,85 g Wasser und 0,5 g pulverförmigem Zeolith (H-ZSM 5) wurden in einem 10 ml Autoklaven gerührt und mit Argon gespült. Dann wurde auf 120°C erhitzt. Nach zwei Stunden wurde auf Raumtemperatur abgekühlt. Die flüssige Phase wurde mit 12 g Aceton verdünnt und nach Abfiltrieren des Zeoliths gaschromatographisch (Kapillarsäule, innerer Standard) analysiert. Sie enthielt 43,9 % Cyclohexylacetat und 21,9 % % Cyclohexanol (bezogen auf eingesetztes Cyclohexen). Die Gehalte an Dicyclohexylether, Cyclohexylbenzol und Acetylbenzol lagen unter der GC-Nachweisgrenze.

### Beispiel 15

2,56 g eines Gemisches aus 32 Gew.-% Cyclohexen, 60 Gew.-% Benzol und 8 Gew.-% Cyclohexan sowie 2,82 g Essigsäure, 0,85 g Wasser und 0,5 g pulverförmigem Zeolith (H-ZSM 10) wurden in einem 10 ml Autoklaven gerührt und mit Argon gespült. Dann wurde auf 120°C erhitzt. Nach zwei Stunden wurde auf Raumtemperatur abgekühlt. Die flüssige Phase wurde mit 12 g Aceton verdünnt und nach Abfiltrieren des Zeoliths gaschromatographisch (Kapillarsäule, innerer Standard) analysiert. Sie enthielt 21,3 % Cyclohexylacetat und 12,2 % Cyclohexanol (bezogen auf eingesetztes Cyclohexen). Die Gehalte an Dicyclohexylether, Cyclohexylbenzol und Acetylbenzol lagen unter der GC-Nachweisgrenze.

### Beispiel 16

2,56 g eines Gemisches aus 32 Gew.-% Cyclohexen, 60 Gew.-% Benzol und 8 Gew.-% Cyclohexan sowie 2,82 g Essigsäure, 0,85 g Wasser und 0,5 g pulverförmigem Zeolith (GA-ZSM 11) wurden in einem 10 ml Autoklaven gerührt und mit Argon gespült. Dann wurde auf 120°C erhitzt. Nach zwei Stunden wurde auf Raumtemperatur abgekühlt. Die flüssige Phase wurde mit 12 g Aceton verdünnt und nach Abfiltrieren des Zeoliths gaschromatographisch (Kapillarsäule, innerer Standard) analysiert. Sie enthielt 33,9 % Cyclohexylacetat und 11,8 % Cyclohexanol (bezogen auf eingesetztes Cyclohexen). Die Gehalte an Dicyclohexylether, Cyclohexylbenzol und Acetylbenzol lagen unter der GC-Nachweisgrenze.

### Vergleichsbeispiel 1

5,0 g eines Gemisches aus 32 Gew.-% Cyclohexen, 60 Gew.-% Benzol und 8 Gew.-% Cyclohexan sowie 5,5 g Essigsäure und 1,0 g Wolframatophosphorsäure (H₃PW₁O₄₀) wurden mit a) 1,5 und b) 3,0 mol Wasser/Mol Cyclohexen versetzt, in einem 10 ml Autoklaven gerührt und mit Argon gespült. Dann wurde auf 120°C erhitzt. Nach zwei Stunden wurde auf Raumtemperatur abgekühlt. Die organische und die wäßrige Phase wurden voneinander getrennt und jeweils die Ausbeuten an Cyclohexylacetat und Cyclohexanol gaschromatographisch (Kapillarsäule, innerer Standard) ermittelt. Durch Zugabe von 12 g Aceton wurden beide Phasen homogenisiert und die Gesamtausbeuten an Cyclohexylacetat und Cyclohexanol bestimmt. Sie betrugen im Fall a) 72 % bzw. 1 % und im Fall b) 37 % bzw. 2 % (bezogen auf eingesetztes Cyclohexen). Die Gehalte an Dicyclohexylether, Cyclohexylbenzol und Acetylbenzol lagen unter der GC-Nachweisgrenze. Weitere Nebenprodukte wurden ebenfalls nicht gefunden.

| Beispiel | organische Phase | | wäßrige Phase | |
|---|---|---|---|---|
| | Ester | Cyclohexanol | Ester | Cyclohexanol |
| | [mol-%]¹⁾ | | [mol-%]¹⁾ | |
| a) | 41,5 | 0 | 29,3 | 1,0 |
| b) | 30,1 | 0,9 | 7,6 | 1,0 |

| | | | | |
|---|---|---|---|---|
| ¹⁾ bezogen auf eingesetztes Cyclohexen. | | | | |

### Vergleichsbeispiel 2

5,0 g eines Gemisches aus 32 Gew.-% Cyclohexen, 60 Gew.-% Benzol und 8 Gew.-% Cyclohexan wurden mit 1,0 g Wolframatophosphorsäure (H₃PW₁₂O₄₀) und mit a) 1, b) 5 und c) 10 Mol Essigsäure/Mol Cyclohexen versetzt, mit Argon gespült und in einem 10 ml Autoklaven gerührt. Dann wurde auf 120°C erhitzt. Nach zwei Stunden wurde auf Raumtemperatur abgekühlt. Die Cyclohexylacetat-Ausbeute wurde gaschromatographisch (Kapillarsäule, innerer Standard) ermittelt und betrug im Fall a) 47 %, im Fall b) 75 % und im Fall c) 85 % (bezogen auf eingesetztes Cyclohexen).

## Patentansprüche

1. Verfahren zur Abtrennung von Cyclohexen aus seinen Gemischen mit Benzol und Cyclohexan, indem man diese Gemische in Gegenwart von festen starksauren Katalysatoren mit Carbonsäuren und Wasser in Berührung bringt, dadurch gekennzeichnet, daß man
(a) die Reaktion in flüssiger Phase bei einer Temperatur im Bereich von 20 bis 180°C und einem Molverhältnis von Carbonsäure zu Wasser im Bereich von 3:15 bis 30:0,1 durchführt, und gegebenenfalls nach der Reaktion mindestens soviel Wasser zusetzt, daß nach der Reaktion eine Trennung in eine wäßrige Phase und eine organische Phase erfolgt,
(b) das in (a) erhaltene flüssige Reaktionsgemisch nach Abtrennen des festen Katalysators in eine wäßrige und eine organische Phase trennt,
(c) die wäßrige Phase ggf. nach Extraktion mit Gemischen aus Benzol, Cyclohexen und/oder Cyclohexan in die Stufe (a) zurückführt, und
(d) die in der organischen Phase enthaltenen Cyclohexylcarbonsäureester verseift und Cyclohexanol gewinnt.

2. Verfahren nach Anspruch 1, dadurch gekennzeichnet, daß man das Molverhältnis von Carbonsäure zu Wasser im Bereich von 3:6 bis 30:0,1 wählt.

## Claims

1. A process for removing cyclohexene from its mixtures with benzene and cyclohexane by contacting these mixtures with carboxylic acids and water in the presence of solid strongly acidic catalysts, characterized in that
(a) the reaction is carried out in liquid phase at a temperature within the range from 20 to 180°C and at a molar ratio of carboxylic acid to water within the range from 3:15 to 30:0.1 and if necessary at least sufficient water is added after the reaction to ensure that, after the reaction, a separation takes place into an aqueous phase and an organic phase,
(b) the liquid reaction mixture obtained in (a) is separated into an aqueous phase and an organic phase after the solid catalyst has been separated off,
(c) the aqueous phase is returned into stage (a) with or without prior extraction with mixtures of benzene, cyclohexene and/or cyclohexane, and
(d) the cyclohexyl carboxylates contained in the organic phase are hydrolyzed to obtain cyclohexanol.

2. A process as claimed in claim 1, characterized in that the molar ratio of carboxylic acid to water is selected within the range from 3:6 to 30:0.1.

## Revendications

1. Procédé de séparation du cyclohexène de ses mélanges avec le benzène et le cyclohexane, conformément auquel on amène ces mélanges en contact avec des acides carboxyliques et de l'eau, en présence de catalyseurs solides fortement acides, caractérisé en ce que
(a) on entreprend la réaction en phase liquide, à une température qui varie de 20 à 180°C et un rapport molaire de l'acide carboxylique à l'eau qui se situe dans la plage de 3:15 à 30:0,1 et on ajoute éventuellement au moins autant d'eau après la réaction qu'il est nécessaire pour que, après la réaction, se produise une séparation en une phase aqueuse et une phase organique,
(b) on sépare le mélange réactionnel liquide obtenu en (a) après la séparation du catalyseur solide en une phase aqueuse et une phase organique,
(c) on renvoie la phase aqueuse, éventuellement après extraction avec des mélanges de benzène, de cyclohexène et/ou de cyclohexane, dans l'étape (a) et
(d) on saponifie l'ester de l'acide cyclohexylcarboxylique obtenu dans la phase organique et on récupère le cyclohexanol.

2. Procédé suivant la revendication 1, caractérisé en ce que l'on choisit le rapport molaire de l'acide carboxylique à l'eau dans la plage de 3;6 à 30:0,1.
